# FASCICULE DE BREVET EUROPEEN

(11) **EP 1 166 746 B1**
(45) Date de publication et mention de la délivrance du brevet: **16.07.2003**
(21) Numéro de dépôt: 01401307.2
(22) Date de dépôt: 18.05.2001
(51) Int. Cl.: A61K 7/00, A61K 7/06, A61K 7/48

(54) **Composition sous forme d'émulsion eau-dans-huile ayant une vitesse de cisaillement évolutive**
Wasser-in-Öl Emulsionzusammensetzung mit verlaufsveränderlicher Schergeschwindigkeit
Water-in-oil emulsion composition with a variable shear rate

(30) Priorité: 22.06.2000 FR 0008011
(43) Date de publication de la demande: 02.01.2002
(73) Titulaire: L'OREAL, 75008 Paris (FR)
(72) Inventeur: Afriat, Isabelle, 75003 Paris (FR)
(74) Mandataire: Rasson, Catherine

(56) Documents cités:
- EP-A- 0 953 336
- EP-A- 0 970 682
- WO-A-97/14401
- US-A- 5 902 569

## Description

L'invention se rapporte à une composition se présentant sous forme d'une émulsion eau-dans-huile (E/H) comportant une forte teneur en eau et un tensioactif siliconé particulier. Cette composition a l'aspect d'un fluide ou d'une crème et est utilisable en particulier dans les domaines cosmétique et/ou dermatologique.

Dans les domaines cosmétique ou dermatologique, il est courant d'utiliser des compositions ayant l'aspect d'un fluide ou d'une crème et constituées d'une émulsion eau-dans-huile (E/H) comportant une phase aqueuse dispersée dans une phase huileuse. Une crème ou un fluide sont dans les domaines considérés, des produits malléables et déformables, par opposition aux compositions solides.

Les émulsions E/H comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches. Toutefois, les émulsions E/H présentent l'inconvénient d'apporter sur la peau à l'application, un toucher assez gras, la phase huileuse étant la phase externe. Ainsi, ces émulsions sont en général utilisées pour les peaux sèches, étant trop grasses pour être utilisées sur les peaux grasses. De plus, les émulsions E/H n'apportent aucune fraîcheur et sont généralement trop riches en huiles pour être utilisées pendant l'été ou dans les pays chauds.

Pour surmonter ces inconvénients, il a été envisagé de préparer des émulsions à forte teneur en eau. Toutefois, la teneur en eau ne peut pas être trop importante pour des raisons de stabilité, ou alors une forte teneur en eau doit être compensée par l'ajout de plusieurs tensioactifs ou d'agents gélifiants qui peuvent nuire au confort de la composition finale et même entraîner des problèmes d'irritations cutanés notamment chez les sujets à peaux sensibles.

On connaît par le document EP-A-970682 des compositions sous forme d'une émulsion eau-dans-huile stable, comportant une quantité importante d'eau et contenant comme agent émulsionnant un diméthicone copolyol comportant des groupes oxyéthylénés et des groupes oxypropylénés. Ces compositions ont une caractéristique rhéologique spécifique qui rend leur utilisation dans le domaine cosmétique, particulièrement intéressante. En effet, lors de l'application sur la peau, elles "cassent", c'est-à-dire qu'elles se fluidifient brutalement sous l'effet du cisaillement, et elles apportent ainsi une très grande fraîcheur sur la peau. De telles compositions sont stables lors de la conservation à une température de 45°C. Toutefois, ces compositions présentent l'inconvénient de ne pas présenter une stabilité satisfaisante dans les cycles de conservation. Un cycle de conservation consiste à faire passer la composition par plusieurs températures sucessives. Ainsi, on maintient la composition un certain temps (par exemple pendant 6 heures) à la température ambiante (environ +20°C), puis pendant la même durée de temps (soit 6 heures), on fait descendre la température jusqu'à environ -20°C, puis on laisse la composition à cette température de -20°C pendant encore la même durée de temps (soit 6 heures), puis on fait remonter la la température jusqu'à la température ambiante (+20°C) pendant la même durée de temps (6 heures), et on répète ceci plusieurs fois (généralement 5 fois). Ce passage à différentes température permet de tester la parfaite stabilité d'une composition. Or, il est avantageux que les compositions cosmétiques présentent une excellente stabilité, quelque soient les conditions dans lesquelles elles se trouvent.

Le document brevet US-A-5 902 569 décrit aux exemples 35 et 36 deux compositions contenant un agent émulsifiant de type diméthicone copolyol non réticulé contenant uniquement des groupes oxyéthylénés. Ces compositions contiennent en plus d'autres agents émulsionnants.

La demanderesse a maintenant trouvé de façon inattendue que les diméthicone copolyols exempts de groupe oxypropyléné, c'est-à-dire ne comportant que des groupes oxyéthylénés, permettaient d'obtenir des émulsions eau-dans-huile contenant une grande quantité d'eau et ayant les mêmes propriétés rhéologiques que celles décrites dans le document EP-A-970682, tout en ayant une excellente stabilité en cycles.

L'invention a pour objet une composition comprenant, dans un milieu physiologiquement acceptable, une phase aqueuse dispersée dans une phase huileuse à l'aide d'un agent émulsionnant siliconé, caractérisée par le fait que la phase aqueuse représente au moins 80 % en poids par rapport au poids total de la composition, que le rapport pondéral phase huileuse/agent émulsionnant est égal ou supérieur à 5 et que l'agent émulsionnant est un diméthicone copolyol comportant uniquement des groupes oxyéthylénés.

On entend par milieu physiologiquement acceptable dans la composition de l'invention, un milieu non toxique et susceptible d'être appliqué sur la peau (y compris l'intérieur des paupières) ou les lèvres d'êtres humains.

La composition obtenue selon l'invention présente généralement une viscosité supérieure à 0,15 Pa.s (1,5 poises) et allant de préférence de 0,2 Pa.s (2 poises) à 20 Pa.s (200 poises). Cette viscosité est mesurée au Rhéomat 180, c'est-à-dire avec l'appareil RM180 Rhéomat de la société METTLER, généralement à une température de 20 à 25°C.

La composition selon l'invention comporte au moins 80 % en poids et de préférence au moins 81 % en poids de phase aqueuse par rapport au poids total de la composition. La phase aqueuse peut constituer jusqu'à 91 % du poids total de la composition.

L'eau constitue au moins 66 % et de préférence au moins 70 % du poids total de la composition.

La phase aqueuse de l'émulsion peut contenir un ou plusieurs alcools inférieurs tels que l'éthanol en une quantité pouvant aller jusqu'à 15 % et mieux jusqu'à 10 % du poids total de la composition. Par ailleurs, cette phase aqueuse peut avantageusement contenir un ou plusieurs polyols tels que la glycérine et les glycols comme le propylène glycol, en une quantité allant par exemple jusqu'à 20 % et mieux jusqu'à 10 % du poids total de la composition.

La composition de l'invention contient comme agent émulsionnant un diméthicone copolyol ne comportant comme groupes oxyalkylénés que des groupes oxyéthylénés, du type methyl(polyoxyéthylène)siloxane. Un tel diméthicone copolyol est exempt de groupes oxypropylénés. Les diméthicone copolyol sont des polymères de polydiméthylsiloxane non réticulés. On peut utiliser notamment dans la composition selon l'invention comme diméthicone copolyol, le produit vendu sous la dénomination commerciale « KF-6015 » par la société Shin-Etsu.

L'agent émulsionnant est présent de préférence en une quantité en matière active allant de 0,5 à 5 % et mieux de 0,6 à 4 % en poids par rapport au poids total de la composition.

De préférence, la composition est exempte d'agents émulsionnnants autres que ceux décrits ci-dessus. Même quand la composition est exempte de tout autre agent émulsionnant, elle présente une excellente stabilité dans le temps.

Le rapport pondéral phase huileuse/agent émulsionnant (en matière active) est égal ou supérieur à 5 et de préférence égal ou supérieur à 8. On entend ici par « phase huileuse » la quantité de tous les constituants huileux, sauf la quantité en matière active, de tensioactif siliconé.

La phase huileuse de la composition selon l'invention peut renfermer toute sorte d'huiles et de corps gras bien connus de l'homme du métier, comme exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

De préférence, la phase huileuse de la composition de l'invention comprend au moins une huile de silicone volatile. La ou les huiles de silicone volatiles peuvent être présentes en une quantité d'au moins 5 % en poids et de préférence allant de 5 à 25 % en poids par rapport au poids total de la composition. Comme huiles de silicone volatiles, on peut citer par exemple les silicones cycliques telles que la pentacyclométhicone, la tétracyclométhicone ou l'hexacyclométhicone. Selon un mode particulier de réalisation de l'invention, la phase huileuse est constituée uniquement d'une ou plusieurs huiles de silicone volatiles.

La phase huileuse peut contenir, en outre d'autres constituants gras tels que les alcools gras comme l'alcool stéarylique, l'alcool cétylique et leur mélange (alcool cétéarylique), et les acides gras.

La composition de l'invention est de préférence exempte de cires.

La phase huileuse est présente dans la composition selon l'invention en une quantité supérieure ou égale à 8,5 % et de préférence de 10 à 18 % en poids par rapport au poids total de la composition.

La composition selon l'invention peut contenir une grande quantité d'électrolyte sans que cela nuise à sa stabilité.

Comme électrolyte, on peut citer par exemple les sels des métaux mono-, di- ou trivalents, et plus particulièrement les sels de métal alcalino-terreux tels que les sels de baryum, de calcium et de strontium ; les sels de métal alcalin tels que les sels de sodium et de potassium, les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les bromures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

De préférence, l'électrolyte est un mélange de sels comprenant notamment des sels de calcium, de magnésium, et de sodium, et notamment un mélange comprenant au moins du chlorure de magnésium, du chlorure de potassium, du chlorure de sodium, du chlorure de calcium, du bromure de magnésium, le dit mélange correspondant à des sels de la mer morte.

La teneur en électrolyte, lorsqu'il est présent, va en général de 0,5 à 20 % et de préférence de 1 à 10 % en poids par rapport au poids total de la composition,

La composition selon l'invention contient un milieu physiologiquement acceptable et peut constituer notamment une composition cosmétique ou dermatologique. Elle trouve son application dans un grand nombre de traitements notamment cosmétiques de la peau, y compris du cuir chevelu, des cheveux, des ongles, et/ou des muqueuses. en particulier pour le soin et/ou le maquillage et/ou la protection solaire de la peau et/ou des muqueuses, ainsi que pour la préparation d'une composition destinée au traitement de la peau, plus particulièrement de la peau grasse (apport de fraîcheur).

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

L'invention a aussi pour objet l'utilisation de la composition telle que définie ci-dessus pour la fabrication d'une composition destinée au traitement des peaux grasses.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme actifs, on peut citer notamment, outre les électrolytes indiqués ci-dessus, les hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines ; l'urée ; les dépigmentants tels que l'acide kojique et l'acide caféique ; les bêta-hydroxyacides tels que l'acide salicylique et ses dérivés ; les alpha-hydroxyacides tels que l'acide lactique et l'acide glycolique ; les rétinoïdes tels que les caroténoïdes ; les filtres, et leurs mélanges.

Le ou les actifs peuvent être par exemple présents en une concentration allant de 0,01 à 20 %, de préférence de 0,1 à 5 % et mieux de 0,5 à 3 % du poids total de la composition.

L'exemple ci-après de composition selon l'invention est donné à titre d'illustration et sans caractère limitatif. Les quantités y sont données en % en poids, sauf mention contraire.

### Exemple : Fluide de soin

### A. Phase huileuse

- Diméthicone copolyol (KF-6015) 1,75 %
- Pentacyclométhicone 17,75 %

### B. Phase aqueuse

- Chlorure de sodium 2,5 %
- Glycérine 7 %
- Eau 71 %

Mode opératoire : on prépare séparément les deux phases et on introduit la phase aqueuse dans la phase huileuse.

On obtient un fluide blanc ayant une viscosité mesurée au RHEOMAT 180, de 2,7 poises (0,27 Pa.s) au temps zéro. Cette viscosité se stabilise après 10 minutes à 2,54 poises (0,254 Pa.s).

Cette composition est stable quand on la passe en cycles sur 5 jours (6 heures à +20°C, 6 heures pour aller jusqu'à -20°C, 6 heures à -20°C, 6 heures pour remonter +20°C, et répétition du cycle 5 fois). Ce passage à différentes température permet de tester la parfaite stabilité d'une composition.

Test comparatif: on remplace dans la composition de l'exemple indiqué ci-dessus le KF-6015 par un tensioactif siliconé comportant des groupes oxyéthylénés et des groupes oxypropylénés, et on les passe en cycles sur 5 jours comme la composition selon l'invention.

| | Exemple selon l'invention | Exemple comparatif 1 | Exemple comparatif 2 |
|---|---|---|---|
| Tensioactif siliconé | KF-6015 | DC2-5185 C (Dow Corning) | Silwet FZ-2108 (Witco) |
| Viscosité (à un temps de 10mn) | 2,54 Poises (0,254 Pa.s) | 18,8 Poises (0,188 Pa.s) | 4,5 Poises (0,45 Pa.s) |
| Stabilité en cycles sur 5 jours | Bonne | Déstabilisation après 2 jours (apparition de gouttes d'huile) | Déstabilisation après 2 jours (déphasage) |
| Stabilité après une semaine à 45°C | Bonne | Bonne | Bonne |

Le DC2-5185 C et le Silwet FZ-2108 utilisés dans les exemples comparatifs sont des diméthicon copolyol comportant à la fois des groupes oxyéthylénés et des groupes oxypropylénés. Le DC2-5185 C comporte 18 groupes oxyéthylénés et 18 groupes oxypropylénés, et le Silwet FZ-2108 comporte un rapport des groupes oxyéthylénés sur les groupes oxypropylénés (33 : 67).

Ce tableau montre que seul le tensioactif siliconé ne comportant que des groupes oxyéthyléné utilisé selon l'invention donne une bonne stabilité en cycles.

## Revendications

1. Composition comprenant, dans un milieu physiologiquement acceptable, une phase aqueuse dispersée dans une phase huileuse à l'aide d'un agent émulsionnant siliconé, **caractérisée par le fait que** la phase aqueuse représente au moins 80 % en poids par rapport au poids total de la composition, que le rapport pondéral phase huileuse/agent émulsionnant est égal ou supérieur à 5 et que l'agent émulsionnant est un diméthicone copolyol non réticulé, comportant uniquement des groupes oxyéthylénés, et en ce que la composition est exempte de tout autre agent émulsionnant.

2. Composition selon la revendication 1, **caractérisée par le fait qu'**elle a une viscosité mesurée au viscosimètre RHEOMAT 180 à un taux de cisaillement de 200 s⁻¹ et à 25 °C, allant de 0,15 Pa.s à 20 Pa.s.

3. Composition selon la revendication 1 ou 2, **caractérisée par le fait qu'**elle comporte au moins 70 % d'eau par rapport au poids total de la composition.

4. Composition selon l'une quelconque des revendications précédentes, **caractérisée par le fait que** l'agent émulsionnant est présent en une quantité allant de 0,5 à 5 % en poids par rapport au poids total de la composition.

5. Composition selon l'une quelconque des revendications précédentes, **caractérisée par le fait que** la phase huileuse est présente en une quantité allant de 10 à 18 % en poids par rapport au poids total de la composition.

6. Composition selon l'une quelconque des revendications précédentes, **caractérisée par le fait que** le rapport pondéral phase huileuse/agent émulsionnant est égal ou supérieur à 8.

7. Composition selon l'une quelconque des revendications précédentes, **caractérisée par le fait que** la phase huileuse comprend une ou plusieurs huiles de silicone volatiles.

8. Utilisation cosmétique de la composition selon l'une quelconque des revendications 1 à 7, pour le traitement, la protection, le soin, le démaquillage et/ou le nettoyage de la peau, des lèvres et/ou des cheveux, et/ou pour le maquillage de la peau et/ou des lèvres.

9. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications 1 à 7.

10. Utilisation de la composition selon l'une quelconque des revendications 1 à 7 pour la fabrication d'une crème destinée au traitement des peaux grasses.

## Patentansprüche

1. Zusammensetzung, die in einem physiologisch akzeptablen Medium eine mit Hilfe eines grenzflächenaktiven Silicons in einer Ölphase dispergierte wässrige Phase aufweist, **dadurch gekennzeichnet, dass** die wässrige Phase mindestens 80 Gew.-%, bezogen auf das Gesamtgewicht der Zusammensetzung, ausmacht, das Gewichtsverhältnis Ölphase/Emulgator mindestens 5 beträgt und der Emulgator ein nicht vernetztes Dimethiconcopolyol ist, das ausschließlich ethoxylierte Gruppen aufweist, und dadurch, dass die Zusammensetzung keinen weiteren Emulgator enthält.

2. Zusammensetzung nach Anspruch 1, **dadurch gekennzeichnet, dass** sie eine mit einem Viscosimeter RHEOMAT 180 bei einer Scherbeanspruchung von 200 s⁻¹ bei 25° C gemessene Viskosität von 0,15 bis 20 Pa·s aufweist.

3. Zusammensetzung nach Anspruch 1 oder 2, **dadurch gekennzeichnet, dass** sie mindestens 70 % Wasser, bezogen auf das Gesamtgewicht der Zusammensetzung, enthält.

4. Zusammensetzung nach einem der vorhergehenden Ansprüche, **dadurch gekennzeichnet, dass** der Emulgator in einer Menge von 0,5 bis 5 Gew.-%, bezogen auf das Gesamtgewicht der Zusammensetzung, vorliegt.

5. Zusammensetzung nach einem der vorhergehenden Ansprüche, **dadurch gekennzeichnet, dass** die Ölphase in einer Menge von 10 bis 18 Gew.-%, bezogen auf das Gesamtgewicht der Zusammensetzung, vorliegt.

6. Zusammensetzung nach einem der vorhergehenden Ansprüche, **dadurch gekennzeichnet, dass** das Gewichtsverhältnis Ölphase/Emulgator mindestens 8 beträgt.

7. Zusammensetzung nach einem der vorhergehenden Ansprüche, **dadurch gekennzeichnet, dass** die Ölphase ein oder mehrere flüchtige Siliconöle enthält.

8. Kosmetische Verwendung der Zusammensetzung nach einem der Ansprüche 1 bis 7 zur Behandlung, zum Schutz, zur Pflege, zum Abschminken und/oder zur Reinigung der Haut, der Lippen und/oder der Haare und/oder zum Schminken der Haut und/oder der Lippen.

9. Verfahren zur kosmetischen Behandlung der Haut einschließlich der Kopfhaut, der Haare und/oder der Lippen, **dadurch gekennzeichnet, dass** auf die Haut, die Haare und/oder die Lippen eine Zusammensetzung nach einem der Ansprüche 1 bis 7 aufgetragen wird.

10. Verwendung der Zusammensetzung nach einem der Ansprüche 1 bis 7 zur Herstellung einer Creme, die zur Behandlung von fettiger Haut vorgesehen ist.

## Claims

1. Composition comprising, in a physiologically acceptable medium, an aqueous phase dispersed in an oily phase with the aid of a silicone emulsifier, **characterized in that** the aqueous phase represents at least 80% by weight relative to the total weight of the composition, **in that** the oily phase/emulsifier weight ratio is greater than or equal to 5 and **in that** the emulsifier is a noncrosslinked dimethicone copolyol comprising only oxyethylenated groups and **in that** the composition does not contain any other emulsifiers.

2. Composition according to Claim 1, **characterized in that** it has a viscosity, measured using a Rheomat 180 viscometer at a shear rate of 200 s⁻¹ and at 25°C, ranging from 0.15 Pa.s to 20 Pa.s.

3. Composition according to Claim 1 or 2, **characterized in that** it comprises at least 70% water relative to the total weight of the composition.

4. Composition according to any one of the preceding claims, **characterized in that** the emulsifier is present in an amount ranging from 0.5% to 5% by weight relative to the total weight of the composition.

5. Composition according to any one of the preceding claims, **characterized in that** the oily phase is present in an amount ranging from 10% to 18% by weight relative to the total weight of the composition.

6. Composition according to any one of the preceding claims, **characterized in that** the oily phase/emulsifier weight ratio is greater than or equal to 8.

7. Composition according to any one of the preceding claims, **characterized in that** the oily phase comprises one or more volatile silicone oils.

8. Cosmetic use of the composition according to any one of Claims 1 to 7, for treating, protecting, caring for, removing make-up from and/or cleansing the skin, the lips and/or the hair, and/or for making up the skin and/or the lips.

9. Cosmetic process for treating the skin, including the scalp, the hair and/or the lips, **characterised in that** a composition according to any one of Claims 1 to 7 is applied to the skin, the hair and/or the lips.

10. Use of the composition according to any one of Claims 1 to 7 to manufacture a cream for treating greasy skin.
